# EUROPÄISCHE PATENTSCHRIFT

(11) **EP 1 713 829 B1**
(45) Veröffentlichungstag und Bekanntmachung des Hinweises auf die Patenterteilung: **16.07.2014**
(21) Anmeldenummer: 05715297.7
(22) Anmeldetag: 11.02.2005
(51) Int. Cl.: G01N 33/68, C07K 16/18

(54) **ANTIKÖRPER ZUR DIAGNOSE UND BEHANDLUNG VON NEUROPSYCHIATRISCHEN ERKRANKUNGEN, INSBESONDERE VON SCHIZOPHRENIE, DEPRESSION UND BIPOLAREN AFFEKTIVEN STÖRUNGEN**
ANTIBODY FOR DIAGNOSING AND TREATING NEUROPSYCHIATRIC DISEASES, IN PARTICULAR SCHIZOPHRENIA, DEPRESSION AND BIPOLAR AFFECTIVE DISORDERS
ANTICORPS DESTINES AU DIAGNOSTIC ET AU TRAITEMENT DE MALADIES NEUROPSYCHIATRIQUES, NOTAMMENT DE LA SCHIZOPHRENIE, DE LA DEPRESSION ET DE TROUBLES AFFECTIFS BIPOLAIRES

(30) Priorität: 13.02.2004 DE 102004007462
(43) Veröffentlichungstag der Anmeldung: 25.10.2006
(73) Patentinhaber: Korth, Carsten, 40219 Düsseldorf (DE)
(72) Erfinder: KORTH, Carsten, 40219 Düsseldorf (DE); LELIVELD, Rutger, S., 40217 Düsseldorf (DE)
(74) Vertreter: Emmel, Thomas
(86) Internationale Anmeldenummer: PCT/EP2005/001371
(87) Internationale Veröffentlichungsnummer: WO 2005/077978

(56) Entgegenhaltungen:
- LAW AMANDA J ET AL: "The distribution and morphology of prefrontal cortex pyramidal neurons identified using anti-neurofilament antibodies SMI32, N200 and FNP7. Normative data and a comparison in subjects with schizophrenia, bipolar disorder or major depression." JOURNAL OF PSYCHIATRIC RESEARCH. 2003 NOV-DEC, Bd. 37, Nr. 6, November 2003 (2003-11), Seiten 487-499, XP002348495 ISSN: 0022-3956
- BERNSTEIN H G ET AL: "Increased number of nitric oxide synthase immunoreactive Purkinje cells and dentate nucleus neurons in schizophrenia." JOURNAL OF NEUROCYTOLOGY. AUG 2001, Bd. 30, Nr. 8, August 2001 (2001-08), Seiten 661-670, XP002348496 ISSN: 0300-4864
- HONER W G ET AL: "MONOCLONAL ANTIBODIES TO STUDY THE BRAIN IN SCHIZOPHRENIA" BRAIN RESEARCH, Bd. 500, Nr. 1-2, 1989, Seiten 379-383, XP002348497 ISSN: 0006-8993
- SIGURDSSON EINAR M ET AL: "Anti-prion antibodies for prophylaxis following prion exposure in mice" NEUROSCIENCE LETTERS, LIMERICK, IE, Bd. 336, Nr. 3, 23. Januar 2003 (2003-01-23), Seiten 185-187, XP002275536 ISSN: 0304-3940
- KORTH C ET AL: "MONOCLONAL ANTIBODIES SPECIFIC FOR THE NATIVE, DISEASE-ASSOCIATED ISOFORM OF THE PRION PROTEIN" METHODS IN ENZYMOLOGY, ACADEMIC PRESS INC, SAN DIEGO, CA, US, Bd. 309, 1999, Seiten 106-122, XP001104847 ISSN: 0076-6879

## Beschreibung

Die vorliegende Erfindung betrifft ein Verfahren zum Gewinnen von Antikörpern und zum Auffinden von Markern zur Diagnose und Behandlung von neuropsychiatrischen Erkrankungen. Im wesentlichen richtet sich die Erfindung auf neuropsychiatrische Erkrankungen wie Schizophrenie, Depression oder bipolare affektive Störungen. Im Rahmen der vorliegenden Anmeldung wird im wesentlichen auf Schizophrenie eingegangen werden. Das hierfür gesagte gilt aber grundsätzlich auch für andere neuropsychiatrische Erkrankungen.

Die Schizophrenie ist eine neuropsychiatrische Erkrankung, die wahrscheinlich heterogene Ursachen hat. Aufgrund von Zwillingsstudien ist inzwischen anerkannt, daß biologische Ursachen für die Entstehung der Schizophrenie verantwortlich sind. Neuropathologisch findet sich bei Patienten mit Schizophrenie eine Erweiterung des dritten Himventrikels, was als unspezifisches Zeichen für einen Hirnstrukturverlust gewertet wird. Die Schizophrenie, zumindest eine Untergruppe der Schizophrenie, nämlich solche mit ausgeprägten sogenannten Negativsymptomen, kann den neurodegenerativen Erkrankungen zugeordnet werden (J. Lieberman 1999, Biological Psychiatry 46: 729f).

Es gibt bislang keine biologische Diagnostik der Schizophrenie oder anderer neuropsychiatrischer Erkrankungen. Zur Diagnosestellung werden von entsprechend ausgebildeten Ärzten (Psychiater, Nervenärzte) bestimmte psychiatrische Kardinalsymptome erfragt; dies kann durch sogenannte Checklisten objektiviert werden. Anders als bei internistischen oder neurologischen Krankheiten kann die Krankheit nicht mit Hilfe von eindeutigen Blut- oder Liquoruntersuchungen oder bildgebenden Verfahren diagnostiziert werden. Dies führt zu einer Unsicherheit in der Diagnostik.

In der Veröffentlichung von "LAW AMANDA J ET AL" (Journal of Psychiatric Research, 2003, 37, Nr. 6 (2003-11), 487-499) sind Antikörper beschrieben, die Neuronen, bzw. Subpopulationen von Neuronen erkennen. Eine sichere Erkennung von Schizophrenie oder überhaupt dem Vorliegen einer Erkrankung ist nicht möglich, da offensichtlich mit den bislang verfügbaren Antikörpern nur eine bestimmte Anzahl der vorhandenen Neuronen-Subpopulationen erkannt werden kann.

In der Veröffentlichung von "BERNSTEIN H G ET AL" (Journal of Neurocytology, Aug. 2001, 30 (2001-8), 661-670) werden Hirnschnitte schizophrener und depressiver Patienten mit Kontrollen aus gesunden Patienten verglichen. Mit einem Antiserum gegen eine Isoform der Stickstoffoxid-Synthase konnten sich bei Schizophrenie-Patienten höhere Signale finden lassen, als bei depressiven bzw. gesunden Patienten. Mit dem fraglichen Antiserum scheint daher eine Diagnose von Schizophrenie möglich zu sein. Aber auch hier ist festzuhalten, dass keine fehlgefalteten Proteine erkannt werden.

Die Veröffentlichung von "HONER W GET AL" (Brain Research, 500, Nr. 1-2, 1989, 379-383) beschreibt die Herstellung monoklonaler Antikörper, die über die Immunisierung von Mäusen mit Homogenaten von Hirnproben schizophrener Patienten gewonnen wurden. Die Ergebnisse zeigen allerdings, dass die so gewonnenen Antikörper noch äußerst unterschiedliche Detektionseigenschaften aufweisen. Eine sichere Differenzierung zwischen Kontrollen und Schizophrenieproben ist, wenn überhaupt, allenfalls mit einigen der gewonnenen Antikörper möglich.

Aus der WO 0026675 ist ein Verfahren zur Diagnose von neuropsychiatrischen Erkrankungen bekannt, bei dem die Anwesenheit von Polyglutamin-haltigen Proteinen in einer Gewebe- oder Körperflüssigkeitsprobe eines Patienten mit Hilfe eines gegen Polyglutamin-haltige Proteindomänen gerichteten Antikörpers getestet wird. Die WO 0026675 führt weiter aus, daß es sich bei den mit diesem Verfahren zu diagnostizierenden neuropsychiatrischen Erkrankungen um Schizophrenie handeln kann.

Polyglutamin-haltige Proteine treten bei einer Vielzahl von neurodegenerativen Erkrankungen auf, so z.B. bei der Huntington'schen Krankheit oder bei spinozerebellären Ataxien. Diese Krankheiten zeichnen sich durch ein mutationsbedingtes erhöhtes Auftreten von sich wiederholenden Glutaminresten in einem oder mehreren Proteinen aus und werden auch als CAG-Repeat-Erkrankungen zusammengefaßt, da das DNA-Triplett CAG für Glutamin codiert.

Dabei führt z.B. bei der Huntington'schen Krankheit eine Mutation im humanen HD-Gen zu einer Erhöhung der Anzahl an Glutaminresten am N-Terminus des Proteins Huntingtin. Das mutante, Polyglutamin-reiche Huntingtin neigt aufgrund seiner fehlerhaften Aminosäuresequenz zur Aggregation mit anderen Polyglutamin-reichen Huntingtin-Molekülen. Dabei bilden sich im Zellkern von Neuronen Agglomerate, die mit dem tödlichen Verlauf der Krankheit einhergehen. Für das Entstehen krankheitsspezifischer Symptome anderer Polyglutamin-Erkrankungen werden ähnliche sequenzbedingte Proteinaggregationen verantwortlich gemacht.

Das aus der WO 0026675 bekannte Verfahren bedient sich eines gegen Polyglutamin-haltige Proteindomänen gerichteten monoklonalen Antikörpers (1C2) und ist daher nicht spezifisch für Schizophrenie. Das Verfahren eignet sich lediglich zum Nachweis von Polyglutamin-haltigen Proteinen. Es bestehen jedoch Zweifel, ob Schizophrenie überhaupt mit dem Auftreten von Polyglutamin-haltigen Proteinen einhergeht. Auf diese Zweifel wird weiter unten ausführlich eingegangen.

Unter der in der WO 0026675 postulierten, allerdings jedoch nicht zutreffenden Prämisse, dass Schizophrenie mit dem Vorhandensein von Polyglutamin-haltigen Proteinen einhergeht, könnte das bekannte Verfahren folglich helfen, einen aufgrund einer psychiatrischen Diagnose gehegten Verdacht auf Schizophrenie zu untermauern. Eine Diagnose der Schizophrenie allein mit diesem Verfahren ist dagegen nicht möglich.

Aufgabe der vorliegenden Erfindung ist es, Antikörper für die Diagnose oder die Behandlung von neuropsychiatrischen Erkrankungen, insbesondere von Schizophrenie, Depresssion oder bipolaren affektiven Störungen zur Verfügung zu stellen. Weitere Aufgabe ist es, ein Verfahren zur Verfügung zu stellen, mit dem die erwähnten neuropsychiatrischen Erkrankungen, insbesondere die Schizophrenie, sicher und eindeutig diagnostiziert werden können. Schließlich ist es eine weitere Aufgabe der vorliegenden Erfindung, pharmazeutische Zusammensetzungen zur Behandlung von z.B. Schizophrenie zur Verfügung zu stellen. Diese Aufgaben werden mit den Merkmalen der vorliegenden Ansprüche gelöst. Erfindungsgemäß ist ein Verfahren zur Gewinnung von Antikörpern vorgesehen, die grundsätzlich fehlgefaltete Proteine erkennrn, die sich einer neuropsychiatrischen Erkrankung zuordnen lassen. Bevorzugt handelt es sich um Antikörper, die fehlgefaltete Proteine erkennen, die spezifisch für Schizophrenie, Depression oder bipolare affektive Störungen sind.

Im Rahmen der Erfindung soll der Begriff neuropsychiatrische Erkrankung insbesondere Psychosen, einschließlich organischer Psychosen, d.h. Erkrankungen, die als Symptom klassische Psychosemerkmale aufweisen (Wahn, Halluzinationen, Gedankenbeeinflussung, Verstimmungen, oder andere affektive Symptome und kognitive Störungen) abdecken. Besonders bevorzugt handelt es sich um Schizophrenie, Depression und bipolare Störungen.

Denkbar ist auch, dass ein erfindungsgemäß gewonnener Antikörper fehlgefaltete Proteine erkennt, die für mehrere neuropsychiatrische Erkrankungen spezifisch sind, wobei die Zuordnung zu einer Erkrankung anhand von weiteren Eigenschaften des Proteins, z.B. seiner Löslichkeit oder seines Molekulargewichts oder aber auch anhand der Herkunft des Proteins aus z.B. einer bestimmten Hirnregion möglich ist.

Erfindungsgemäß ist vorgesehen, dass der Antikörper durch Immunisierung von geeigneten Tieren mit aufgereinigten Hirnfraktionen von Patienten gewonnen wird, die an einer neuropsychiatrischen Erkrankung leiden, wobei während der Aufreinigung Schritte vorgesehen sind, die eine Anreicherung von fehlgefalteten Proteinen bewirken.

Um den erfindungsgemäß gewonnenen Antikörper näher zu erläutern, muß zunächst noch einmal auf die bereits erwähnten Polyglutamin-Erkrankungen zurückgekommen werden. Da diese wie oben geschildert auf eine Mutation in einem Gen zurückgehen, ist die Prädisposition für diese Erkrankungen in der Regel erblich. Sie werden daher auch unter dem Begriff "hereditäre neurodegenerative Erkrankungen" zusammengefaßt. Es ist jedoch umstritten, ob es sich z.B. bei Schizophrenie überhaupt um eine Polyglutamin-Erkrankung handelt, da bislang im Gegensatz zur Huntington'schen Krankheit kein Polyglutamin-haltiges Protein identifiziert werden konnte, das mit der Entstehung der Schizophrenie in Verbindung gebracht werden oder bei Schizophrenie-Patienten in anomalen Konzentrationen nachgewiesen werden kann.

Polyglutaminerkrankungen sind überdies seltene Erkrankungen. Im Verhältnis zur Huntington'schen Krankheit treten neurodegenerative Erkrankungen, die nicht mit Polyglutamin korreliert sind, wesentlich häufiger auf, die Alzheimersche Erkrankung z.B. 150 mal, die Parkinsonsche Krankheit 30 mal und die Schioziphrenie 100 mal häufiger (S. Prusiner 2001, New England Journal of Medicine 344: 1516f).

Zwar ist anerkannt, daß es eine genetische Disposition für Schizophrenie gibt, jedoch scheinen die Ursachen multifaktoriell zu sein. Hierfür spricht, daß selbst der eineiige Zwilling eines an Schizophrenie erkrankten Patienten nur ein 50-%iges Risiko hat, ebenfalls an Schizophrenie zu erkranken (I. Gottesman and J. Shields 1982, Schizophrenia: the epigenetic puzzle, Cambride University Press, Cambridge; Kendler et al 1993, The Roscommon Family Study. I. Methods, diagnosis of probands, and risk of schizophrenia in relatives, Arch Gen Psychiatry 50 (7): 527).

Die Erfinder haben nun erstmals Hinweise dafür gefunden, daß das Vorhandensein von fehlgefalteten Proteinen als diagnostischer Marker für Schizophrenie oder andere neuropsychiatrische Erkrankungen, wie z.B. Depression oder bipolare affektive Störungen dienen kann. Dabei wurde postuliert, daß die Fehlfaltung auf posttranslationale Ursachen zurückzuführen ist und nicht - oder nur zu einem geringen Teil - mit sequenzbedingten Anomalien wie CAG-Repeats in Verbindung gebracht werden kann. Verantwortlich für die Fehlfaltung kann z.B. ein Fehler während der Proteinbiosynthese oder Proteinprozessierung im Endoplasmatischen Retikulum sein, der z.B. durch fehlerhafte Prozessierungsenzyme wie z.B. Transloconkomponenten oder Chaperone verursacht werden kann. Ebenso ist denkbar, daß Reparaturenzyme oder Proteasen, die bereits fehlgefaltete Proteine reparieren bzw. verdauen sollen, fehlerhafte Funktionen aufweisen. Es ist auch denkbar, daß bestimmte Enzyme der posttranslationalen Verarbeitung oder Proteasen ein zunächst richtig gefaltetes Protein fehlerhaft derart modifizieren, dass es übermässig in einer fehlerhaften Faltung produziert wird.

Die Fehlfunktionen der Prozessierungsenzyme, Reparaturenzyme und/oder Proteasen können dabei gegebenenfalls durch Mutationen in den jeweiligen Genen oder deren Interaktionspartner auf Proteinebene verursacht sein, was eine eventuelle familiäre Disposition für Schizophrenie erklären könnte. Als mögliche Kandidaten sind dabei die Gene für die Proteine DISC-1, Dysbindin, Neuregulin 1, COMT, RGS4, der metabotrope Glutamatrezeptor-3, DAAO, and G72 im Gespräch (P. Harrison & M. Owen 2003, Lancet 361:417f; P. Harrison and D. R. Weinberger 2005, Molecular Psychiatry 10:40-68).

Erfindungsgemäß werden die Hirnfraktionen von z.B. Schizophreniepatienten gezielt aufgereinigt, um die posttranslational fehlgefalteten Proteine zu isolieren und anzureichern. In einer bevorzugten Ausgestaltung können mit den aufgereinigten Fraktionen in herkömmlicher Weise geeignete Tiere (Kaninchen, Mäuse, Schafe, Hühner) immunisiert und so Antikörper gewonnen werden, die spezifisch an diese Proteine binden.

Anstelle einer Immunisierung ist es möglich, mit rekombinanten Liganden- oder Antikörperbibliotheken, die z.B. in Phagen exprimiert werden, geeignete Antikörper oder deren Fragmente zu identifizieren, die spezifisch an die aufgereinigten, fehlgefalteten Proteine binden, und die nach Identifizierung durch Mutagenese *in vitro* affinitäts-maturiert werden können.

Erfindungsgemäß ist dabei vorgesehen, dass der Aufreinigung ein Reinigungsschritt mit ionischen Detergenzien ist. Die Aufreinigung der Hirnfraktionen mit ionischen Detergenzien dient dazu, leicht denaturierbare Proteine in der aufzureinigenden Probe aufzulösen. Fehlgefaltete Proteine, die zur Aggregation neigen, werden dagegen durch ionische Detergenzien insbesondere bei Temperaturen zwischen 0 und 10 Grad Celsius nicht denaturiert, verbleiben daher nicht in Lösung, pelletieren also unter (Ultra-) Zentrifugationsbedingungen und können so in nativer Form für die Immunisierung isoliert werden.

Um auszuschließen, daß es sich bei den schizophreniespezifischen Proteinen um Polyglutamin-haltige Proteine handelt, hat der Anmelder erfindungsgemäß isolierte Hirnfraktionen von Schizophreniepatienten sowie gesunden Testpersonen einem Western Blot mit dem markierten Antikörper MW1 unterzogen. Dieser Antikörper erkennt ähnlich wie der in der WO 0026675 verwendete Antikörper 1C2 Polyglutamin-reiche Epitope in Proteinen bzw. Polyglutamin-Polymere. In diesen Versuchen stellte sich heraus, daß sowohl in den Hirnfraktionen von Schizophreniepatienten als auch in denen gesunder Testpersonen Polyglutamin-haltige Proteine nachweisbar waren. Es waren jedoch keine Unterschiede im Polyglutamin-Gehalt feststellbar. Die Ergebnisse werden weiter unten besprochen. Der Anmelder hat daraus gefolgert, daß Polyglutamin-haltige Proteine bei Schizophreniepatienten nicht in erhöhter Konzentration auftreten und daher nicht als diagnostische Marker für Schizophrenie verwendet werden können.

Wie bereits erläutert, dient die Aufreinigung der Hirnfraktionen mit ionischen Detergenzien dazu, mißgefaltete Proteine, die durch diese Detergenzien nicht denaturiert werden, zu isolieren. Überdies ist vorgesehen, dass der Reinigungsschritt bei 0 - 10 °C durchgeführt wird, da die mißgefalteten Proteine bei höheren Temperaturen auch durch ionische Detergenzien denaturiert werden können. Besonders bevorzugt wird bei diesem Reinigungsschritt eine Temperatur von bei 0 - 5°C verwendet.

In einer weiteren bevorzugten Ausgestaltung der Erfindung ist vorgesehen, dass das während des Reinigungsschritts verwendete ionische Detergens in einer Konzentration zwischen 0.2 und 2 % eingesetzt wird. Auch diese Maßnahme dient dazu, eine Denaturierung der mißgefalteten Proteine zu verhindern, da diese bei höheren Konzentrationen auch durch ionische Detergenzien denaturiert werden können. Bevorzugt wird bei diesem Reinigungsschritt eine Konzentration verwendet, bei der gewährleistet ist, daß das Detergens noch keine Mizellen ausbildet ("critical micellar concentration", CMC).

Unterhalb der CMC binden Detergensmoleküle an Proteine, während sie oberhalb der CMC Mizellen ausbilden. Eine Konzentration unterhalb der CMC ist für die Aufreinigung von fehlgefalteten Proteinen günstiger, da dann vermehrt Detergens an unerwünschte Proteine bindet und diese im Gegensatz zu den fehlgefalteten Proteinen in Lösung bringt. Die CMC unterscheidet sich von Detergens zu Detergens. Es ist außerdem zu berücksichtigen, dass die CMC auch vom pH-Wert und der Temperatur des Mediums abhängt. Bevorzugt wird bei diesem Reinigungsschritt eine Konzentration zwischen 0.2 und 1 % verwendet.

In einer weiteren bevorzugten Ausgestaltung ist vorgesehen, dass das während des Reinigungsschritts verwendete ionische Detergens Sarkosyl ist. Sarkosyl wird deswegen bevorzugt verwendet, da dies ermöglicht, einerseits genügend unerwünschte, korrekt gefaltete Proteine zu denaturieren, andererseits aber die Mikroaggregate der fehlgefalteten Proteine zu erhalten. Diese Aggregations-/Löslichkeitsbalance hängt von der CMC eines Detergens ab.

Das Detergens Sodium dodecyl sulfat (SDS) wirkt dagegen bespielsweise zu stark denaturierend und ist daher für den genannten Zweck weniger geeignet.

Besonders bevorzugt wird Sarkosyl in einem Konzentrationsbereich zwischen 0.3 und 0.42 % verwendet. Unter Normalbedingungen erreicht Sarksyl bei 0.45 % seine CMC.

Während des Reinigungsschritts wird besonders bevorzugt ein Ultrazentrifugationsschritt bei mindestens 100 000 x g eingesetzt. Da die fehlgefalteten Proteine unter den erwähnten Bedingungen nicht löslich sind, finden sie sich nach der Ultrazentrifugation im Pellet, während andere Proteine im Überstand bleiben.

In einer weiteren bevorzugten Ausgestaltung ist vorgesehen, daß während der Autceinigung ein Reinigungsschritt mit ß-Faltblatt-bindenden Substanzen wie Kongorot, Thioflavin oder ß-Faltblatt-bindenden Peptiden vorgesehen. Diese Substanzen können ggf. in einer Chromatographie-Säule ö.ä. immobilisiert sein. Da die Sekundärstruktur der fehlgefalteten, schizophreniespezifischen Proteine einen erhöhten Anteil an ß-Faltblatt-Domänen ("betasheet") aufweist, können die gesuchten Proteine auf diese Weise gezielt angereichert werden.

Ebenso ist bevorzugt vorgesehen, dass während der Aufreinigung ein Protease-Verdauungsschritt bei einer Temperatur von 0 - 10 °C vorgesehen ist. Dieser Schritt eignet sich ebenso zur Anreicherung von fehlgefalteten, schizophreniespezifischen Proteinen, da diese aufgrund ihrer Fehlfaltung unter kalten Bedingungen eine erhöhte Resistenz gegen Proteasen wie z.B. Proteinase K aufweisen, während nicht fehlgefaltete Proteine bei diesen Temperaturen durch Proteasen verdaut werden.

Bevorzugt ist der erfindungsgemäß gewonnene Antikörper ein monoklonaler Antikörper. Für die Gewinnung monoklonaler Antikörper wird zunächst ein geeignetes Tier immunisiert, und dann in bekannter Weise antikörperproduzierernde Zellen (z.B. B-Zellen der Milz) aus dem immunisierten Tier entnommen, mit immortalisierten Myelomzellen fusioniert und selektioniert. Die erhaltenen Hybridomazellen werden dann in Hinblick auf die Spezifität der von Ihnen produzierten Antikörper gegen das fehlgefaltete Protein ausgewählt. Anstelle einer Immunisierung ist es möglich, mit rekombinanten Liganden- oder Antikörperbibliotheken, die z.B. in Phagen exprimiert werden, geeignete Antikörper zuidentifizieren, die spezifisch an die aufgereinigten, fehlgefalteten Proteine binden, und die nach Identifizierung durch Mutagenese *in vitro* affinitäts-maturiert werden können.

Insbesondere können im Rahmen der Erfindung monoklonale Antikörper zum Einsatz kommen, die die Bezeichnungen 7B2 und 9C9 tragen. Hybridomazellen, mit denen die Anitkörper produziert werden können wurden unter den Nummern DSM ACC2713 und DSM ACC2714 gemäß Budapester Vertrag hinterlegt.

Monoklonale Antikörper ermöglichen eine höhere Spezifität der immunochemischen Nachweisreaktion und verbessern damit die Genauigkeit z.B. eines Nachweisverfahrens. Besonders bevorzugt ist dabei vorgesehen, daß der Antikörper ein konformations-spezifischer monoklonaler Antikörper ist, ein Antikörper also, der ein Epitop eines gegebenen Proteins nur in einer bestimmten Konformation erkennt, z.B. ausschließlich dann, wenn das Epitop in einer ß-Faltblatt-Konformation vorliegt.

Denkbar ist auch, daß der Antikörper ein rekombinanter Antikörper ist. Für die Gewinnung eines solchen Antikörpers wird z.B. aus den Milzzellen immunisierter Tiere DNA isoliert und daraufhin die Paratop-kodierenden cDNA-Fragmente kloniert.

Es kann auch vorgesehen sein, dass der Antikörper ein hirngängiger Antikörper ist. Unter dem Begriff "hirngängig" wird verstanden, daß die Antikörper die Blut-Hirn-Schranke überwinden können. Dabei sind verschiedene Möglichkeiten denkbar, die Blut-Hirn-Schranke für die Antikörper passierbar zu machen, etwa durch gleichzeitige Gabe gegeigneter Pharmazeutika oder hypertonischer Zuckerlösungen. Andererseits können die Antikörper auch durch molekularbiologische Modifikationen in die Lage versetzt werden, die Blut-Hirn Schranke passieren zu können, z.B. in dem ihre Hydrophobizität erhöht oder ihr Molekulargewicht erniedrigt wird oder die Antikörper mit einer Signalsequenz markiert werden, die deren gezielten Transport über die Blut-Hirn-Schranke fördert.

Monoklonale Antikörper weisen eine sehr viel höhere Spezifität auf als polyklonale Antikörper, bergen aber im therapeutischen Einsatz ebenso wie erstere die Gefahr von Abstoßungsreaktionen. Denkbar ist daher daher, dass der Antikörper ein chimärer oder ein humanisierter Antikörper ist. Bei chimären Antikörpern werden auf molekularbiologischem Wege die konstanten Domänen z.B. von Mausantikörpern durch die entsprechenden konstanten Domänen menschlicher Antikörper ersetzt. Zusätzlich werden bei humanisierten Antikörpern auch die Grundgerüste der variablen Domäne durch entsprechende menschliche Sequenzen ersetzt, so daß nur die für die Antigenbindung verantwortlichen hypervariablen Regionen weiterhin murinen Ursprungs sind. Solchermaßen modifizierte Antikörper verursachen bei der Applikation im Patienten nur sehr geringe und in der Regel tolerierbare Abstoßungsreaktionen.

Weiterhin denkbar ist, dass der Antikörper ein Antikörperfragment ist. Hierbei kann es sich zum Beispiel um monovalente F(ab)-Fragmente handeln, wie sie z.B. nach Papain-Verdau von IgG-Antikörpermolekülen gewonnen werden, oder um bivalente F(ab)₂-Fragmente, wie sie z.B. nach Trypsin-Verdau gewonnen werden. Antikörperfragmente lassen sich leichter in chimäre Antikörper klonieren oder mit humanisierten oder humanen Sequenzen bzw. mit Signalsequenzen kombinieren. Außerdem verursachen Antikörperfragmente eine schwächere Abstoßungsreaktion.

In einer weiteren Ausgestaltung kann vorgesehen sein, dass die erfindungsgemäßen Antikörper an eine pharmazeutisch aktive Substanz und/oder gemäß noch einer weiteren Ausgestaltung an ein Isotop oder ein radioaktiv markiertes Molekül gekoppelt sind. Die letztgenannten Antikörper könnten z.B,. bei der Radioimmuntherapie oder der nuklearmedizinischen Diagnostik Anwendung finden.

Weiterhin ist ein Verfahren zur Diagnose von z.B. Schizophrenie oder Depression oder bipolaren affektiven Störungen mittels Antikörpern, die an für neuropsychiatrische Erkrankungen spezifische Proteine binden, vorgesehen. Dabei werden die Antikörper mit einer Gewebe- oder Körperflüssigkeitsprobe eines Patienten in Kontakt gebracht und gegebenenfalls gebildete Antikörper-Proteinkomplexe nachgewiesen. Das eventuelle Vorhandensein von Antikörper-Proteinkomplexen wird dabei als positiver Befund für Schizophrenie, Depression, oder Bipolare affektive Erkrankung gewertet. Das Verfahren ist dadurch gekennzeichnet, dass einer der zuvor beanspruchten Antikörper verwendet wird.

Dabei hat sich herausgestellt, daß die von dem Anmelder erfindungsgemäß hergestellten Antikörper spezifisch an z.B. schizophrenietypische Proteine binden. Um dies zu belegen, hat der Anmelder immunochemische Vergleichsversuche mit Hirnhomogenaten gesunder und schizophreniekranker Testpersonen durchgeführt. Dabei wurden Hirnfraktionen von Schizophreniepatienten sowie gesunden Testpersonen einem Western Blot mit den erfindungsgemäß gewonnenen Antikörpern unterzogen. Im Ergebnis fand der Anmelder immunoreaktive Banden, die nur in den Hirnfraktionen von Schizophreniepatienten nachweisbar waren. Die erfindungsgemäß gewonnenen Antikörper erkennen also Proteine, die nur bei Schizophreniepatienten auftreten. Die Ergebnisse werden weiter unten besprochen.

In einer bevorzugten Ausgestaltung des Verfahrens ist vorgesehen, dass das Vorhandensein von Antikörper-Proteinkomplexen mittels ELISA, Western-Blot oder immungekoppelter Fluoreszenzmethoden nachgewiesen wird. Es ist jedoch auch jedes andere geeignete Verfahren, mit dem die Bindung von Antikörpern bzw. anderen Sondenmolekülen an Antigene nachgewiesen werden kann, denkbar.

Da fehlgefaltete Proteine bei neuropsychiatrischen Erkrankungen, insbesondere Schizophrenie, aber auch Depression oder der bipolaren affektiven Erkrankung vermutlich schon lange vor Ausbruch der Krankheit in Gewebe- oder Körperflüssigkeitsproben eines Patienten nachweisbar sind, eignet sich das erfindungsgemäße Verfahren auch dazu, eine gegebenenfalls vorhandene Disposition für die jeweilige neuropsychiatrische Erkrankung, also für Schizophrenie, aber auch Depression oder der bipolaren affektiven Erkrankung nachzuweisen. In einer bevorzugten Ausgestaltung des erfindungsgemäßen Verfahrens ist daher vorgesehen, dass der positive Befund eine diagnostizierte Prädisposition und/oder eine positive Diagnose für die spezifische neuropsychiatrische Erkrankung, im speziellen also der Schizophrenie oder eine Untergruppe der Schizophrenie, bzw. der Depression oder bipolar affektiven Erkrankung ist.

In einer besonders bevorzugten Ausgestaltung des erfindungsgemäßen Verfahrens ist vorgesehen, dass die zu untersuchende Körperflüssigkeitsprobe Liquor, Urin, Blut oder Serum ist. Es ist davon auszugehen, daß z.B. bei einem Patienten mit Verdacht auf Schizophrenie, aber auch solchen mit Verdacht auf Depression oder eine bipolar affektive Erkrankungkrankheitssspezifische fehlgefaltete Proteine außer in der Hirnmatrix auch in Körperflüssigkeiten vorkommen. Dies gilt insbesondere für den Liquor (Spinalflüssigkeit), der in ständiger Verbindung mit dem Gehirn steht, und von dem in der klinischen Routine durch Punktion des Rückenmarkskanals gefahr- und schmerzlos Proben genommen werden können.

Die vorliegende Erfindung beschränkt sich jedoch nicht nur auf die Diagnose von Schizophrenie, einer Depression oder einer bipolaren affektiven Störungen, sondern erstreckt sich auch auf die Verwendung z.B. der erfindungsgemäßen Antikörper bei der Herstellung einer pharmazeutischen Zusammensetzung zur Behandlung dieser Erkrankungen.

Es handelt sich dabei um Zusammensetzungen, die einem Patienten insbesondere in hirngängiger Form appliziert werden können. Insbesondere werden die zuvor beanspruchten Antikörper verwendet mit dem Zweck, dass die ins Gehirn gelangten Antikörper dort an z.B. schizophrenie-spezifische, fehlgefaltete Proteine binden und z.B. deren Aggregation mit anderen fehlgefalteten Proteinen verhindern.

In einer besonders bevorzugten Ausgestaltung ist vorgesehen, dass die applizierten Antikörper mit pharmazeutisch aktiven Substanzen gekoppelt sind. Bei diesen Substanzen kann es sich z.B. um Marker handeln, die das vom Antikörper gebundene Protein dergestalt markieren, daß es von einer Protease verdaut oder von einer Mikrogliazelle phagozytiert wird. Es kann sich dabei aber auch um Substanzen handeln, die die von den Antikörpern markierten Proteine für bildgebende Verfahren (NMR, CT) sichtbar machen. Denkbar ist auch, dass mit radioaktiv markierten Substanzen gekoppelte Antikörper eingesetzt werden.

Denkbar wäre auch die Herstellung von pharmazeutischen Zusammensetzungen unter Verwendung von immunogenen Substanzen, die eine Immunantwort evozieren, dergestalt dass das Immunsystem des Patienten Antikörper gegen fehlgefaltete, z.B. schizophreniespezifische Proteine ausbildet.

Bei diesen Substanzen kann es sich z.B. um schizophreniespezifische Proteine oder Proteine, die spezifisch für andere neuropsychiatrische Erkrankungen, wie der Depression oder bipolar affektiven Erkrankung sind, handeln, wie sie mit einem der zuvor beanspruchten Aufreinigungsverfahren isoliert werden, oder deren rekombinant produzierten Äquivalente. Um diesen Proteinen ihre gegebenenfalls vorhandene Pathogenizität zu nehmen, ist es dabei unter Umständen erforderlich, sie vor der Applikation euiner geeigneten Behandlung zu unterziehen. Ebenso kann es sich bei diesen Substanzen z.B auch um Fragmente dieser über die zuvor beanspruchten Antikörper definierten, für Schizophrenie (oder ggf. Depression oder bipolare affektive Erkrankung) spezifischen Proteine handeln, die lediglich noch die immunogenen Bereiche enthalten, aber nicht mehr pathogen sind.

Die Erfindung soll im folgenden anhand von Beispielen verdeutlicht werden:

### A: Isolierung fehlgefalteter Proteine

### Verwendete Lösungen oder Puffer (sterilfiltriert):

VRL-Puffer: 50mM HEPES, pH 7,5, 250mM Sucrose, 5mM MgCl₂, 100mM KCH₃COO, 2mM PMSF, Proteaseinhibitor-Tabletten, "Complete EDTA-free"

### (Roche 1873580)

High-Sucrose-Puffer: 50mM HEPES, pH 7,5, 1,6M Sucrose, 100mM KAc (KCH3COO), 0,5% Triton-X-100, 1mM PMSF#
High-Salt-Puffer: 50mM HEPES, pH 7,5, 1M NaCl, 10mM MgCl2, 100 U/ml DNAse I
Sarkosyl-Puffer: 50mM HEPES, pH 7,5, 0,5% Sarkosyl

### Weitere Puffer:

A. 50 mM HEPES pH 7.5, 300 mM NaCl, 250 mM sucrose, 5 mM EDTA, 5 mM GSH, 1 % NP-40, 0.2% sarkosyl.
B. 50 mM HEPES pH 7.5, 1.5 M NaCl, 250 mM sucrose, 5 mM EDTA, 5 mM GSH, 1% NP-40.
C. 50 mM Tris pH 8, 250 mM sucrose, 5 mM MgCl₂, 5 mM GSH, 1 % NP-40.
D. 50 mM HEPES pH 7.5, 5 mM EDTA, 5 mM GSH, 1 % NP-40.
E. 50 mM HEPES pH 7.5, 2.3 M sucrose, 5 mM EDTA, 5 mM GSH, 1 % NP-40. Adjust sucrose concentration using Puffer D.
F. 50 mM HEPES pH 7.5, 150 mM NaCl, 250 mM sucrose, 5 mM EDTA, 5 mM GSH, 2x PIs.

Abkürzungen: Pis = protease inhibitor cocktail (Roche); PMSF = phenylmethylsulfonyl fluoride (Sigma), GSH = reduced glutathione (Sigma)

### 1. Protokoll "Herstellung der unlöslichen Proteinfraktion (fehlgefaltetes Proteom) aus einem 10%igen Hirnhomogenat"

1. Es werden schockgefrorene Hirnproben der Hirnregionen BA8, BA9 und BA23, BA24 von verstorbenen Patienten, bei denen zu Lebzeiten eine Schizophrenie diagnostiziert wurde verwendet. BA8, BA9, BA23 und BA24 beziehen sich auf sogenannte Brodmannsche Areale und entsprechen bestimmten Neuronenzentren im Neokortex.
2. Gehirnprobe auf Trockeneis einwiegen und in entsprechendem Volumen VRL-Puffer homogenisieren. Aufbewahrung bei -80°C.
3. Homogenat auf Eis auftauen und mit 0,5% Triton-X-100 in 2ml-Mikroreaktionsgefässen bei 20000g und 4°C 20 min zentrifugieren. Überstand sammeln und Pellet erneut im gleichen Volumen VRL-Puffer mit Triton resuspendieren.
4. Zweite Zentrifugation wie oben. Überstand zum ersten Überstand geben, davon 500µl separat bei -80°C aufbewahren.
5. Pellets in insgesamt 4ml High-Sucrose-Puffer lösen. Ultrazentrifugation in Ultra-Clear Centrifuge Tubes 5ml bei 130000g, 4°C, 45min (Beckmann MLS-50, 40000rpm) Überstand sammeln, überstehende Lipidschicht in separatem Mikroreaktionsgefäß einfrieren.
6. Pellet wieder in 4ml High-Sucrose-Puffer resuspendieren und erneut zentrifugieren wie oben. Überstand zum ersten Überstand geben. Aufbewahrung bei -20°C
7. Pellet in 4ml High-Salt-Puffer lösen, Inkubation über Nacht bei 4°C. Ultrazentrifugation in Ultra-Clear Centrifuge Tubes 5ml bei 130000g, 4°C, 45min (Beckmann MLS-50, 40000rpm ). Überstand sammeln und Pellet erneut in High-Salt-Puffer (ohne DNAse ) resuspendieren. Falls erforderlich, Pellet dazu in einer Insulinspritze mit 0.6mm bis 0.4mm Kanüle aufnehmen.
8. Zweite Zentrifugation wie oben. Überstand zum ersten Überstand geben, davon 500µl separat bei -80°C aufbewahren. Aufbewahrung bei -20°C
9. Pellet in 200µl Sarkosyl-Puffer lösen. Dazu Pellet zunächst in 100µl Puffer mit der Pipettenspitze zerkleinern und in ein 0,5ml-Mikroreaktionsgefäß überführen. Gefäss und Pipettenspitze mit weiteren 100µl Sarcosyl-Puffer spülen und zu den ersten 100µl dazugeben. Mit Insulinspritze und 0.4mm Kanüle das Pellet lösen. Bei 4°C ca. 1 Stunde im Rotator inkubieren. Evtl. nach der Hälfte der Zeit erneut mit einer Insulinspritze homogenisieren.
10. Ultrazentrifugation in Mikroreaktionsgefäss (Beckmann 357448 Polyallomer Tubes with Snap-on Caps), deren Gewicht auf einer Analysenwaage bestimmt wurde, Bei 112000g, 4°C, 45min) (Beckmann TLA-55, 50000rpm). Überstand sammeln und erneut mit 200µl Sarkosyl-Puffer waschen. Resuspension und Zentrifugation wie oben.
11.Überstand sammeln und Gewicht der Pellets bestimmen. Aufbewahrung bei -80°C.

### 2. Protokoll "Herstellung der unlöslichen Proteinfraktion (fehlgefaltetes Proteom) aus einem 10%igen Hirnhomogenat"

1. Das Hirnstück (z. B. 0.3-0.4 gr = 1 vol) bei 5 % (w/v) in Puffer A (plus 2x PIs, 1mM PMSF) homogenisieren und bei 1800 x g, 30 min, 4°C zentrifugieren. Das Pellet wird in Puffer A gewaschen und weiterverarbeitet (5 ml [15 vol] Waschvolumen).
2. Das Pellet in Puffer B suspendieren (10 ml [30 vol] Waschvolumen; zudem noch 1 mM PMSF und 0.2% Sarkosyl hinzugefügen), zentrifugieren (1800 x g, 30 min, 4°C) und einmal in Puffer B waschen (5 ml [15 vol] Waschvolumen).
3. Das Pellet wird anschliessend mit Puffer C gewaschen weiterverarbeitet (5 ml [15 vol] Waschvolumen). dann noch einmal gründlich resuspendiert in Puffer C (5 ml [15 vol] Waschvolumen; plus 2x PIs, 1 mM PMSF, Benzonase and DNaseI, 40 U/ml jeweils) und für 30 min bei 37 °C geschüttelt. Anschliessend wird die gleiche Mischung über Nacht bei 4 °C geschüttelt und am nächsten Morgen zentrifugiert (1800 x g, 30 min, 4°C).
4. Das Pellet wird anschliessend in Puffer A (plus 1 mM PMSF, 5 ml [15 vol] Waschvolumen) gewaschen, zentrifugiert (1800 x g, 30 min, 4°C) und in Puffer D resuspendiert. Nach erfolgreicher Resuspension wird Sucrose dazugetan so dass eine Sucroseendkonzentration von 1.6 M erreicht wird (70% der Sucrosekonzentration von Puffer E).
5. Die Suspension von 4) wird in einem 4:1 Verhältnis auf ein Kissen von 1 ml Puffer E gelegt und mit der Ultrazentrifuge bei 45.000 rpm im MLS-50 Rotor für 45 Minuten zentrifugiert (ungefähr 120.000 x g).
6. Die Interphase zwischen den Sucrosephasen wird abpipettiert (ungefähr 1 ml) und im Verhältnis 1:4 mit Puffer D verdünnt. Diese Suspension wird erneut auf 1 ml 70%igen Puffer E gelegt und zentrifugiert bei 45.000 rpm im MLS-50 Rotor für 45 Minuten zentrifugiert (ungefähr 120.000 x g). Das resultierende Pellet (0.1 ml) wird in Puffer F aufgenommen.
7. Das resultierende Pellet ("unlösliche Proteinfraktion) wird anschliessend sowohl zur Immmunisierung als auch für die Dot Blots und SDS-PAGE/Western blot verwendet.

Alle Schritte werden bei 4°C, bzw. auf Eis durchgeführt. Bei den Schritten 1.-4. wird in einer Tischzentrifuge bei 1800 x g, 30 Minuten bei 4 Grad Celsius zentrifugiert.

### B: Herstellung von Antikörpern

### a) Polyklonale Antikörper

Hühner, Kaninchen und Mäuse (BALB/c) werden mit ca. 500-1000 µg/100µl von vier Schizophreniepatienten gepoolten Pellets immunisiert. Dabei wurde dem (aggregierten) Antigen RIBI (Sigma) als Adjuvans zugegeben. Die Tiere werden dann zweimal im Abstand von 3 Wochen geboostet. Zwei Wochen nach der letzten Boosterung wird die Immunantwort untersucht. Bei Hühnern werden jeweils eine Woche nach der Boosterung, angefangen nach der ersten Boosterung, Eier gesammelt und aus dem Eigelb Antikörper (IgY) nach Standardmethoden isoliert.

### b) Monoklonale Antikörper

Für die Gewinnung monoklonaler Antikörper wird wie beschrieben ein geeignetes Tier immunisiert, und dann in bekannter Weise (G Köhler, C Milstein 1975, Continuos cultures of fused cells secreting antibody of predefined specificity. Nature, 256, 495-497) antikörperproduzierernde Zellen (z.B. B-Zellen der Milz) aus dem immunisierten Tier entnommen, mit immortalisierten Myelomzellen fusioniert und selektioniert. Die erhaltenen Hybridomazellen werden dann in Hinblick auf die Spezifität der von ihnen produzierten Antikörper gegen das fehlgefaltete Protein ausgewählt.

### Die monoklonalen Antikörper 7B2 und 9C9 wurden wie folgt hergestellt:

Unlösliche, fehlgefaltete Proteine gereinigt aus gefrorenen Hirnstücken (Kortex, BA8) von 15 Patienten mit Schizophrenie wurden gepoolt und in Prionprotein (PrP) knockout Mäuse zur Immunisierung unter Verwendung von RIBI als Adjuvant subkutan injiziert. PrP knockout Mäuse wurden verwendet, da diese bereits erfolgreich zum Erzeugen von konformationsspezifischen monoklonalen Antikörpern verwendet wurden und in dieser Erfindung auch bevorzugt für die Erzeugung von konformationsspezifischen mABs gegen andere Antigene als PrP verwendet werden. Die Mäuse wurden zweimal geboostert, nach jeweils drei Wochen Abstand; zehn Tage nach dem letzten Booster wurden die Mäuse an zwei aufeinanderfolgenden Tagen intraperitoneal geboostert und am dritten Tag die Milz zur Fusion entnommen. Die Fusion der Milzzellen (Splenozyten) mit den Myelomzellen zu resultierenden Hybridomzellen erfolgte nach Standardmethoden.

### C: Immunologische Charakterisiserung

Generell wurden mit den gewonnenen Antikörpern Hirnhomogenate normaler, schizophrener, depressiver, und bipolar affektiv erkrankter Patienten per Western Blot bzw. DotBlot untersucht.

### Die Ergebnisse sind in den Figuren wiedergegeben.

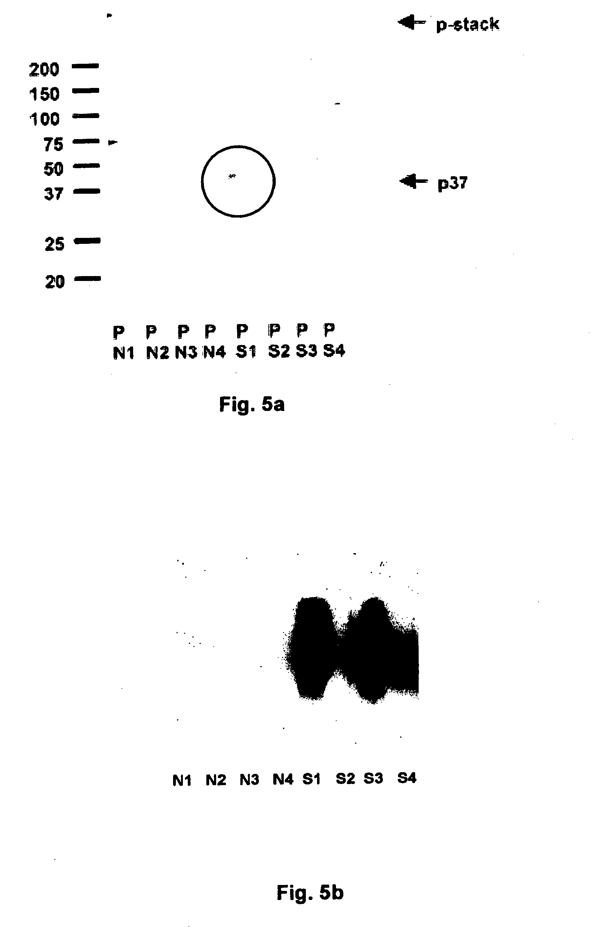

Dabei zeigt:

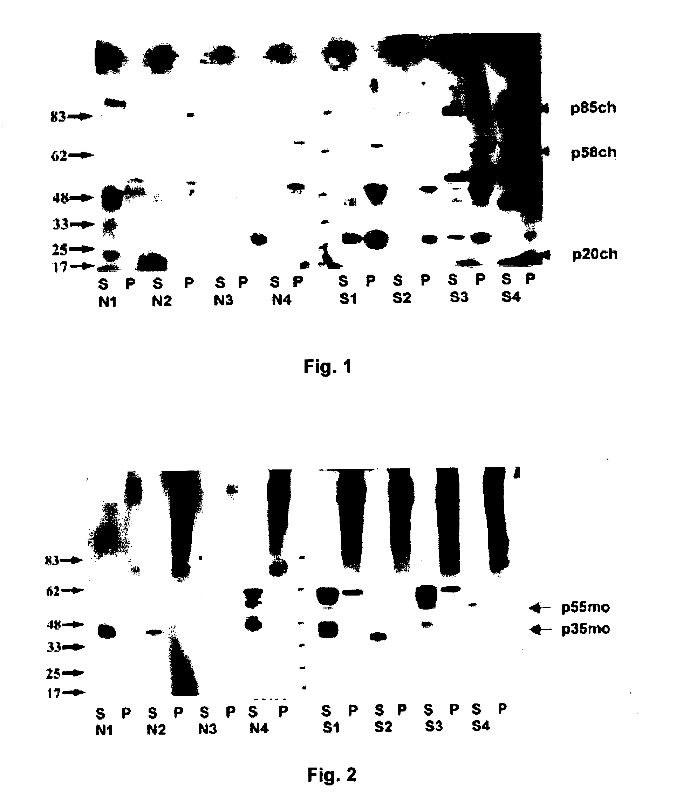

Fig. 1: einen Western blot von biochemisch fraktioniertem Pellet oder 1. Überstand (nach Sarkosylinkubation) von Hirnhomogenaten Normaler oder Schizophrener Patienten nach biochemischer Fraktionierung auf schwer lösliche, sarkosylresistente Proteinaggregate (Antikörper: Hühner-IgY).
   S = 1. Überstand, P = Pellet; N1 - N4 = Hirn gesunder Personen, S1 - S4 = Hirn von Schizophrenie-Patienten. Die Pfeile (p85ch, p58ch, p20ch) beziehen sich auf immunoreaktive Banden, die spezifisch nur bei Schizophreniepatienten auftreten, und somit biologische Marker darstellen. Die Pfeile auf der linken Seiten geben Molekulargewichte an.
Fig. 2: einen Western blot von biochemisch fraktioniertem Pellet oder 1. Überstand von Hirnhomogenaten Normaler oder Schizophrener Patienten nach biochemischer Fraktionierung auf schwer lösliche, sarkosylresistente Proteinaggregate. (Antikörper: Mausserum).
   S= 1. Überstand, P= Pellet; N1 - N4 = Hirn gesunder Personen, S1 - S4 = Hirn von Schizophrenie-Patienten. Die Pfeile beziehen sich auf immunoreaktive Banden, die spezifisch nur bei Schizophreniepatienten auftreten, und somit biologische Marker darstellen (p55mo, p35mo). Die Pfeile auf der linken Seiten geben Molekulargewichte an.;
Fig. 3a: einen Dotblot mit monoklonalem Antikörper RC1 unter nicht denaturierenden Bedingungen von einem Pool von Sarkosyl-resistentem Pellet (Fraktion X) von gepooltem Normalhirn (N; BA9) und gepooltem Schizophreniehirn (S; BA9).
   Als Antikörper wurde der monoklonale konformationsspezifische Antikörper RC1 verwendet, der die native Oberflächenstruktur eines Proteins, das spezifisch bei Schizophrenie vorhanden ist, mit hoher Konformationsspezifität erkennt;

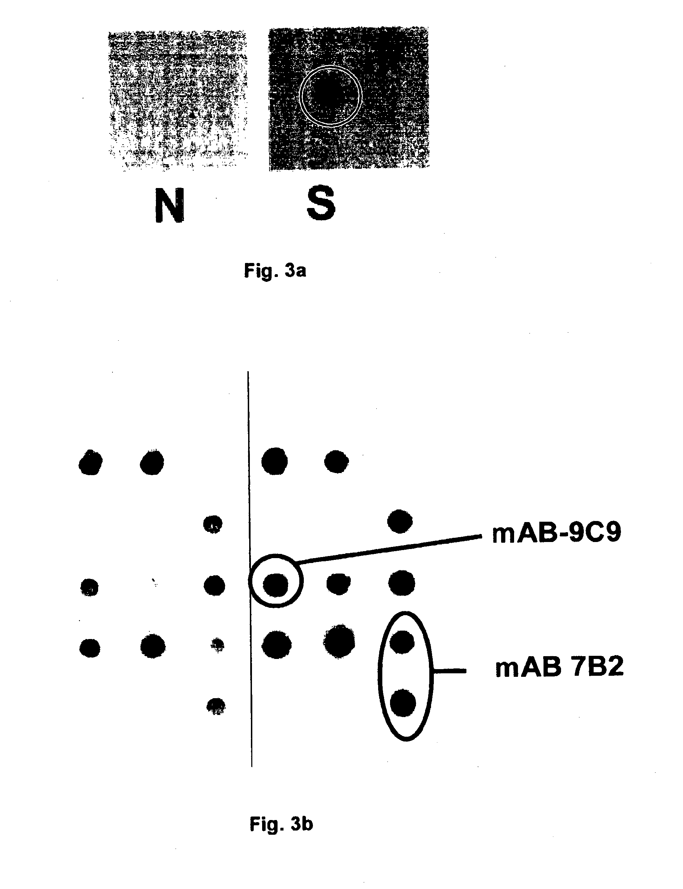

Fig. 3b: Dot Blot Assay mit monoklonalen Antikörpern 7B2 und 9C9. Die monoklonalen Antikörper 7B2 und 9C9 wurden nach folgendem Screening erhalten und getestet: Die Zellkulturüberstände von ca. 3000 Hybridomzellen wurden mit einem Dotblotassay in einem speziellen Apparat (ELIFA Apparat; Pierce, USA) im 96 Wellformat gescreent. Hierbei wurden parallel gleiche Mengen der gepoolten unlöslichen Proteine (aufgereinigt nach Protokoll 1) von Patienten mit Schizophrenie und Normalpersonen auf Nitrozellulose aufgetragen, die Membran wurde geblockt mit 5% non-fat dry milk in TBST (Tris-gepufferte Salzlösungen enthaltend Tween) und die Zellkulturüberstände in den Wells des ELIFA-Apparats auf den Dots für 2 h bei Raumtemperatur inkubiert. Die Membran wurde gewaschen und mit einem Zweitantikörper (anti-mouse IgG/M), an den kovalent Peroxidase gekoppelt ist für eine Stunde inkubiert. Der Blot wurde anschliessend gewaschen, mit ECL Substrat versetzt, und auf Hyperfilm (Amersham) entwickelt. Die Ergebnisse zeigt Fig. 3b. Von denjenigen Überständen, die sehr viel stärker oder ausschliesslich mit den unlöslichen Proteinen der Schizophreniehirne reagieren (schwarz), wurden die Hybridomzellen gepickt und mehrfach subkloniert. Anschliessend wurde in grösseren Mengen Überstand in serumfreiem Medium (PFHM; Gibco, USA) produziert, das dann für anschliessende Untersuchungen verwendet wurde. Als besonders geeignet erwiesen sich zwei Antikörper, die mit 7B2 und 9C9 bezeichnet wurden.

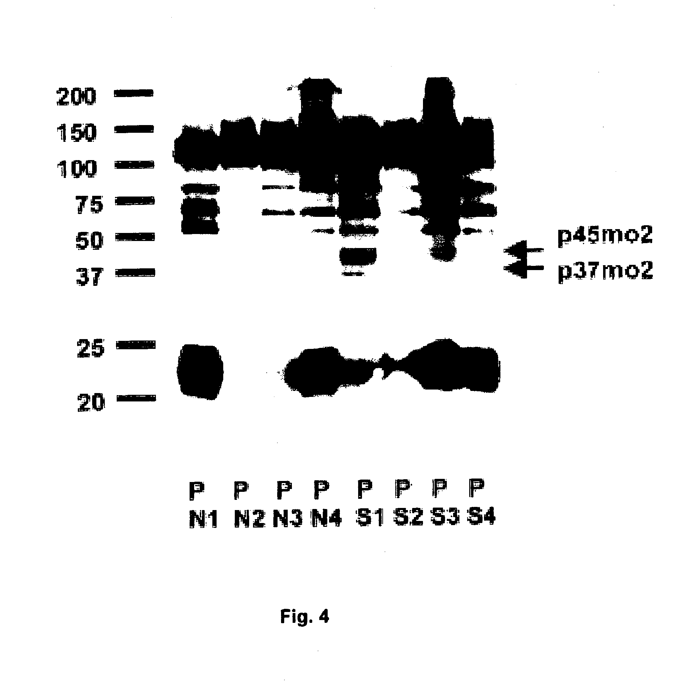

Fig. 4: einen Western blot von biochemisch fraktioniertem Pellet von Hirnhomogenaten Normaler oder Schizophrener Patienten.
   Die Homogenate wurden nach dem 1. Protokoll aufgereinigt. Antikörper stammen aus Maus-Antiserum. P = Pellet; N1 - N4 = Hirn gesunder Personen, S1-S4 = Hirn von Schizophrenie-Patienten. Die Pfeile beziehen sich auf immunoreaktive Banden, die spezifisch nur bei Schizophreniepatienten auftreten, und somit biologische Marker darstellen (p45mo2, p37mo2); Antikörper: Mausserum
Fig. 5: a) einen Western blot von biochemisch fraktioniertem Pellet von Hirnhomogenaten Normaler oder Schizophrener Patienten.
   Die Homogenate wurden nach dem 1. Protokoll aufgereinigt. Als Antikörper wurde der monoklonale AK SX16.3 verwendet. P = Pellet; N1 - N4 = Hirn gesunder Personen, S1 - S4 = Hirn von Schizophrenie-Patienten. Die Pfeile beziehen sich auf immunoreaktive Banden, die spezifisch nur bei Schizophreniepatienten auftreten, und somit biologische Marker darstellen (p37, p-stack). p-stack ist eine Immunoreaktivität aus dem Well. Es handelt sich hierbei um unlösliche Proteine, die beim Beschicken des Gels mit aufgenommen wurden, aber aufgrund ihrer Unlöslichkeit nicht im Gel transportiert wurden. Ein Teil von p-stack ist in Lösung gegangen und bildet p37;
Fig. 5 b.: :Einen Western blot von biochemisch fraktioniertem Pellet von Hirnhomogenaten Normaler oder Schizophrener Patienten aus der Region BA9.
   Die Homogenate wurden nach dem 1. Protokoll aufgereinigt. Als Antikörper wurde der monoklonale AK 7B2 verwendet. P = Pellet; N1 - N4 = Hirn gesunder Personen, S1 - S4 = Hirn von Schizophrenie-Patienten. Es ist zu sehen, dass AK 7B2 nur Immunoreaktivität bei Schizophreniehirnen zeigt.

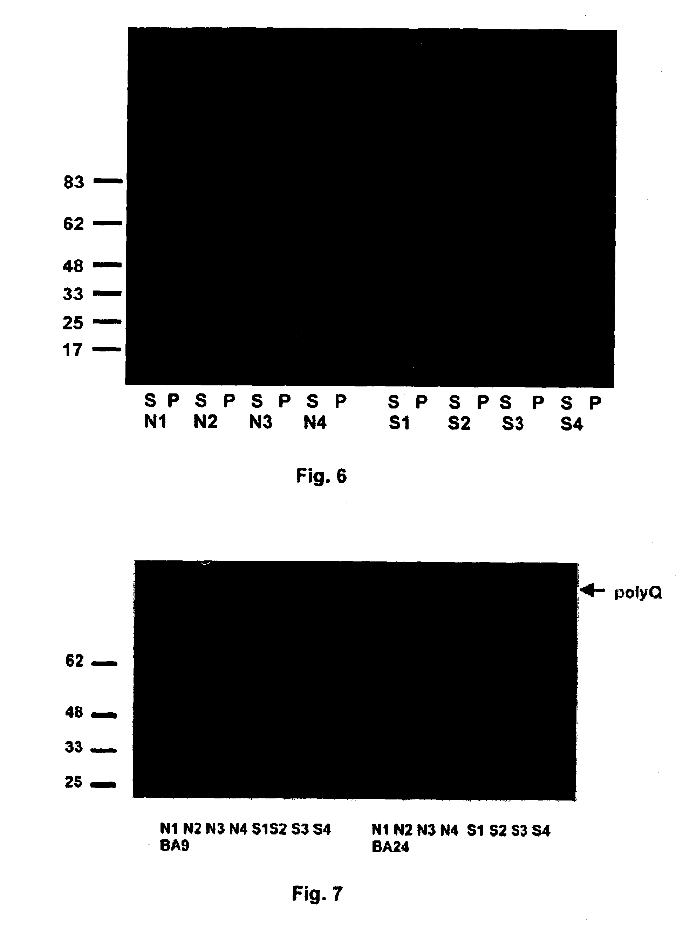

Fig. 6: Western blot von biochemisch fraktioniertem Pellet bzw. dem 1. Überstand von Hirnhomogenaten Normaler oder Schizophrener Patienten.
   Die Homogenate wurden nach dem 1. Protokoll aufgereinigt. Antikörper stammen aus Kaninchen-Antiserum. S = 1.Überstand, P = Pellet; N1 - N4 = Hirn gesunder Personen, S1 - S4 = Hirn von Schizophrenie-Patienten. Obwohl es keine immunoreaktiven Banden gibt, die ausschliesslich bei Schizophrenen, aber nicht bei gesunden Personen auftreten, ist deutlich zu sehen, dass im Bereich der markierten Rechtecke nur bei Schizophrenen Immunoreaktivität vorhanden ist. Das bedeutet, dass in dem molekularen Bereich unterhalb von 60kD ausschliesslich bei Schizophrenen unlösliche Proteine pelletieren;
Fig. 7: Western blot von biochemisch fraktioniertem Pellet bzw. dem 1. Überstand von Hirnhomogenaten Normaler oder Schizophrener Patienten.
   Die Homogenate wurden nach dem 1. Protokoll aufgereinigt. Als Antikörper wurde der monoklonale Antikörper: MW1, verwendet, der Polyglutamin-haltige ("polyQ") Epitope erkennt [Ko et al., 2001, Brain Research Bulletin 56:319f] S = 1.Überstand, P = Pellet; N1 - N4 = Hirn gesunder Personen, S1 - S4 = Hirn von Schizophrenie-Patienten. BA9 und BA24 bezeichnen verschiedene Hirnregionen nach Brodman. Man erkennt Polyglutamin-haltige Banden am oberen Rand des Gels, bei denen es sich um im Well pelettierte, SDS-resistente, polyglutamin-Multimere handelt, die beim Beschicken des Gels mit aufgenommen wurden, aber aufgrund ihrer relativen Unlöslichkeit nicht im Gel transportiert wurden. Es ist jedoch kein Unterschied zwischen Normalen und Schizophrenie-Patienten festzustellen. Es liegt also ein Hinweis dafür vor, daß Polyglutamin-haltige Proteine nicht Schizophrenie-spezifisch sind.

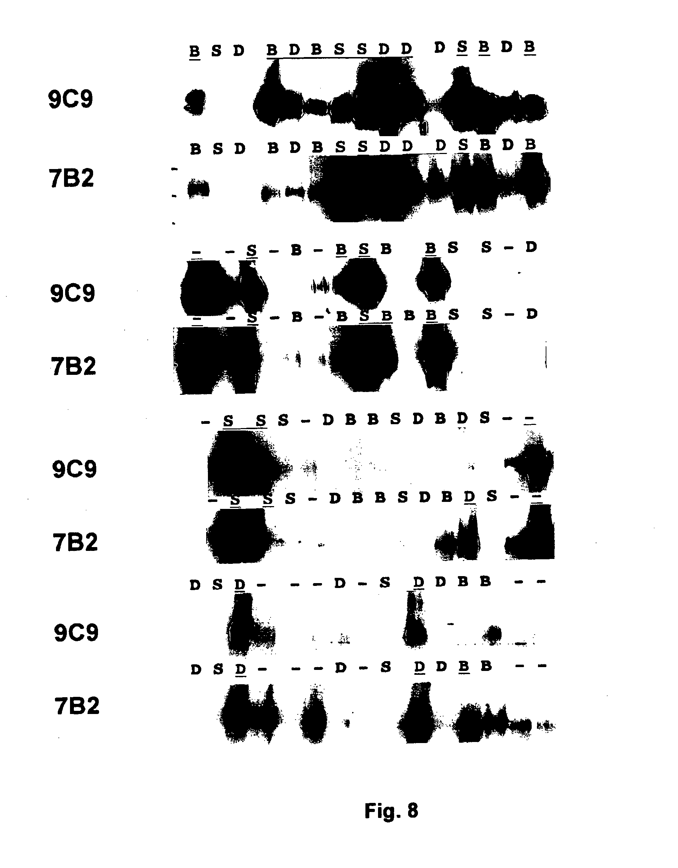

Fig. 8: Western Blot mit 7B2 und 9C9 gegen unlösliche Proteinfraktionen B8 der SMRI Consortium Collection (SMRI).
   Die monoklonalen Antikörper 7B2 und 9C9 wurden gegen die unlöslichen Proteinfraktionen von BA8 Hirnhomogenaten von Patienten mit Schizophrenie (dieselben, die zum Immunisieren genommen worden waren), Depression, Bipolar disorder, und Normalkontrollen auf dem Western blot (WB) getestet. Die Ergebnisse zeigt Fig. 8. Die unlösliche Proteinfraktion der einzelnen Hirne wurde mittels SDS-PAGE aufgetrennt und im WB dargestellt. Die Bewertung erfolgte blind, d.h. ohne dass die oben angegebenen Diagnosen der einzelnen Hirne bekannt waren. Positiv bewertet wurden nur solche Banden, die ausreichende Immunreaktivität besassen.
   Für mAB 9C9 ergab sich nach statistischer Auswertung mittels crosstabs / Pearson Chi-Square Analyse, dass die als positiv bewerteten immunoreaktiven Banden folgendes erkannten: 2 (von 15) Normalkontrollen; 5 (von 15) Depressive, 7 (von 15) Bipolare, und 7 (von 15) Schizophrene. Damit ergab sich eine signifikante Erkennung von 9C9 von krank vs. normal (p= 0.042), schizophren vs. normal (p=0.046), und bipolar vs. normal (p=0.046). Die statistische Analyse wurde mit SPSS (version 11.0 auf Apple G4) durchgeführt.
   Für mAB 7B2 ergab sich nach statistischer Auswertung mittels crosstabs / Pearson Chi-Square Analyse, dass die als positiv bewerteten immunoreaktiven Banden folgendes erkannten: 2 (von 15) Normalkontrollen; 6 (von 15) Depressive, 5 (von 15) Bipölare, und 7 (von 15) Schizophrene. Damit ergab sich eine signifikante Erkennung von 7B2 von schizophren vs. normal (p=0.046), aber nicht bipolar vs. normal (p=0.195) oder krank vs. normal (p=0.058). Die statistische Analyse wurde mit SPSS (version 11.0 auf Apple G4) durchgeführt.

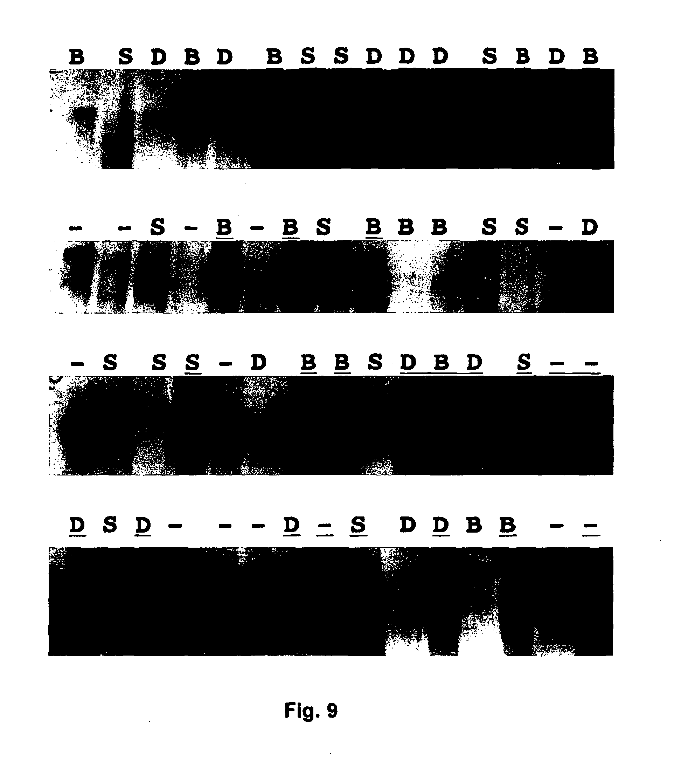

Fig. 9: Western Blot von 7B2 gegen unlösliche Proteinfraktionen von BA23 (SMRI)
   Weiterhin wurde 7B2 gegen die unlöslichen Proteinfraktionen von BA23 Hirnhomogenaten, also einer anderen Hirnregion, von Patienten mit Schizophrenie, Depression, Bipolar disorder, und Normalkontrollen auf dem Western blot getestet (Bild 3). Positiv bewertet wurden nur solche Banden, die ausreichende Immunreaktivität besassen und insbesondere die zweite, etwas kleinere Bande einschlossen.

Für mAB 7B2 ergab sich nach statistischer Auswertung mittels crosstabs / Pearson Chi-Square Analyse, dass die als positiv bewerteten immunoreaktiven Banden folgendes erkannten: 4 (von 15) Normalkontrollen; 9 (von 15) Depressive, 10 (von 15) Bipolare, 4 (von 15) Schizophrene. Damit ergab sich eine signifikante Erkennung von 7B2 von bipolar vs. normal (p=0.028), aber nicht von Schizophrenie vs. normal (p=1) oder krank vs. normal (p=0.09). Die statistische Analyse wurde mit SPSS (version 11.0 auf Apple G4) durchgeführt.

Im Vergleich der Daten aus Fig. 8 und Fig. 9 zeigt sich also, dass das 7B2 Antigen zwar in der BA8 Region (präfrontaler Cortex) unlöslich ist, und hier auch Schizophrenie-Hirne von Normalhirnen unterscheiden kann, dass dies für eine andere Hirnregion (BA23, hinteres Cingulum) nicht zutrifft. Hier kann 7B2 nicht mehr Schizophrenie-Hirne von Normalhirnen unterscheiden, dafür aber Bipolar-Hirne von Normalhirnen, was er wiederum in der BA8 Region nicht konnte.

Diese Ergebnisse spiegeln die Tatsache wieder, dass es in der biologischen Ursache der Schizophrenie und der Bipolar Disorder Überlappungen gibt, die sich in der unterschiedlichen Löslichkeit des 7B2 Antigens in verschiedenen Hirnregionen zeigen. Diese Unterschiede könnnen ihre Ursache in unterschiedlicher Präsenz von bestimmten Zelltypen oder eines bestimmten extrazellulären Milieus haben.

Zusammenfassend lässt sich feststellen, dass mAB 7B2 und 9C9 zur spezifischhen Erkennung neuropsychiatrischer Erkrankungen, speziell der Schizophrenie, der Bipolaren Störung, und der Depression anhand von Hirnhomogenaten geeignet sind.

### Hinterlegung von biologischem Material:

Hybridomazellen, die die Antikörper 7B2 und 9C9 produzieren wurden gemäß Budapester Vertrag bei der Deutschen Sammlung von Mikroorganismen und Zellkulturen GmbH, Mascheroder Weg 1b, D38124 Braunschweig wie folgt hinterlegt:
1. Hybridomazellen (Antikörper 7B2): DSM ACC2713, Hinterlegungsdatum: 26.01.2005.
2. Hybridomazellen (Antikörper 9C9): DSM ACC2714, Hinterlegungsdatum: 26.01.2005.

## Patentansprüche

1. Verfahren zum Gewinnen von Antikörpern die fehlgefaltete Proteine erkennen, die sich spezifisch einer neuropsychiatrischen Erkrankung wie Schizophrenie, Depression oder bipolare affektive Störungen zuordnen lassen, bei dem
- Hirnfraktionen von Patienten mit einer neuropsychiatrischen Erkrankung gezielt zur Anreicherung posttranslational fehlgefalteter Proteine aufgereinigt werden, wobei während der Aufreinigung ein Reinigungsschritt in Gegenwart von ionischen Detergenzien bei 0-10 C° durchgeführt wird und eine Pelletierung der nicht denaturierten fehlgefalteten Proteine unter Zentrifugationsbedingungen erfolgt, und
- mit den aufgereinigten Fraktionen Antikörper hergestellt werden,
- und daraus die Antikörper ausgewählt werden, die spezifisch an die fehlgefalteten Proteine in den aufgereinigten Fraktionen binden.

2. Verfahren gemäß Anspruch 1, bei dem das während des Reinigungsschritts verwendete ionische Detergens in einer Konzentration zwischen 0.2 und 2 % eingesetzt wird.

3. Verfahren gemäß der Ansprüche 1 oder 2, bei dem das während des Reinigungsschritts verwendete ionische Detergens Sarkosyl ist.

4. Verfahren gemäß der Ansprüche 1-3, bei dem der Reinigungsschritt mit einem ionischen Detergens einen Ultrazentrifugationsschritt bei mindestens 100 000 x g umfaßt.

5. Verfahren gemäß der Ansprüche 1-4, bei dem während der Aufreinigung ein Reinigungsschritt mit ggf. immobilisierten, ß-Faltblatt-bindenden Substanzen wie Kongorot, Thioflavin oder ß-Faltblatt-bindenden Peptiden vorgesehen ist.

6. Verfahren gemäß der Ansprüche 1-5, bei dem während der Aufreinigung ein Protease-Verdauungsschritt bei einer Temperatur von 0 - 10 °C vorgesehen ist.

7. Verfahren zum Auffinden von Markern für neuropsychiatrische Erkrankungen wie Schizophrenie, Depression oder bipolare affektive Störungen, bei dem
- Hirnfraktionen von Patienten mit einer neuropsychiatrischen Erkrankung gemäß Anspruch 1 gezielt zur Anreicherung posttranslational fehlgefalteter Proteine aufgereinigt werden,
- mit den aufgereinigten Fraktionen Antikörper hergestellt werden, die spezifisch an Proteine in den aufgereinigten Fraktionen binden,
- und in Hirnhomogenaten mit den Antikörpern Proteine erkannt werden, die nur bei erkrankten Patienten auftreten.

8. Verwendung von in Hirnfraktionen von Patienten mit einer neuropsychiatrischen Erkrankungen gemäss Anspruch 1 angereicherten und dann aus den Hirnfraktionen isolierten postranslational fehlgefalteten Proteinen als diagnostische Marker für neuropsychiatrische Erkrankungen.

9. Antikörper herstellbar gemäß einem der Ansprüche 1-6, mit der Bezeichnung 7B2, der mit Hybridomazellen produzierbar ist, die unter der Nummer DSM ACC2713 hinterlegt sind, zur Diagnose oder Behandlung von Erkrankungen, insbesondere von neuropsychiatrischen Erkrankungen wie Schizophrenie, Depression oder bipolare affektive Erkrankung.

10. Antikörper herstellbar gemäß einem der Ansprüche 1-6, mit der Bezeichnung 9C9, der mit Hybridomazellen produzierbar ist, die unter der Nummer DSM ACC2714 hinterlegt sind, zur Diagnose oder Behandlung von Erkrankungen, insbesondere von neuropsychiatrischen Erkrankungen insbesondere von neuropsychiatrischen Erkrankungen wie Schizophrenie, Depression oder bipolare affektive Erkrankung.

11. Verwendung von mit dem Verfahren gemäß der Ansprüche 1-6 hergestellten Antikörpern bzw. Antikörpern gemäß der Ansprüche 9 oder 10 zur Herstellung einer einem Patienten in hirngängiger Form applizierbaren pharmazeutischen Zusammensetzung zur Behandlung der neuropsychiatrischen Erkrankungen Schizophrenie, Depression oder bipolare affektive Erkrankung.

12. Verwendung nach Anspruch 11, wobei die Antikörper mit pharmazeutisch aktiven Substanzen gekoppelt sind.

13. Verwendung von fehlgefalteten Proteinen, die sich durch ein Verfahren isolieren lassen, bei dem
- Hirnfraktionen von Patienten mit einer neuropsychiatrischen Erkrankung gezielt zur Anreicherung posttranslational fehlgefalteter Proteine aufgereinigt werden, wobei während der Aufreinigung ein Reinigungsschritt in Gegenwart von ionischen Detergenzien bei 0-10 C° durchgeführt wird und eine Pelletierung der nicht denaturierten fehlgefalteten Proteine unter Zentrifugationsbedingungen erfolgt, und die von den gemäss Anspruch 1 hergestellten Antikörpern erkannt werden, zur Herstellung einer pharmazeutischen Zusammensetzung zur Behandlung der neuropsychiatrischen Erkrankungen Schizophrenie, Depression oder bipolare affektive Erkrankung.

14. Verwendung gemäß Anspruch 13 , wobei sich die fehlgefalteten Proteine einer der Erkrankungen Schizophrenie, Depression oder bipolare affektive Erkrankung zuordnen lassen.

## Claims

1. Process for obtaining antibodies which recognize misfolded proteins which can be assigned specifically to a neuropsychiatric disorder such as schizophrenia, depression or bipolar affective disorders, in which process
- brain fractions of patients with a neuropsychiatric disorder are purified in a targeted manner so as to concentrate post-translationally misfolded proteins, where during the purification process a purification step is carried out in the presence of ionic detergents at 0-10°C and the non-denatured misfolded proteins are pelleted under centrifugation conditions, and
- antibodies are raised using the purified fractions,
- from which those antibodies are selected which specifically bind to the misfolded proteins in the purified fractions.

2. Process according to Claim 1, in which the ionic detergent used during the purification step is employed at a concentration of between 0.2 and 2%.

3. Process according to Claims 1 or 2, in which the ionic detergent used during the purification step is sarkosyl.

4. Process according to Claims 1-3, in which the purification step with ionic detergent includes an ultracentrifugation step at at least 100 000 x g.

5. Process according to Claims 1-4, in which a purification step with optionally immobilized, β-sheet-binding substances such as Congo red, thioflavin or β-sheet-binding peptides is provided during the purification process.

6. Process according to Claims 1-5, in which a protease digestion step at a temperature of 0-10°C is provided during the purification process.

7. Method of identifying markers for neuropsychiatric diseases such as schizophrenia, depression or bipolar affective disorders, in which process
- brain fractions of patients with a neuropsychiatric disorder according to Claim 1 are purified in a targeted manner to concentrate post-translationally misfolded proteins,
- antibodies which specifically bind to proteins in the purified fractions are raised using the purified fractions,
- and, using the antibodies, proteins are recognized in brain homogenates which only occur in diseased patients.

8. Use as diagnostic markers for neuropsychiatric disorders of post-translationally misfolded proteins which have been concentrated in brain fractions of patients with a neuropsychiatric disorder according to Claim 1 and which have then been isolated from the brain fractions.

9. Antibody which can be raised according to one of Claims 1 to 6, with the name 7B2, which can be produced using hybridoma cells which have been deposited under the number DSM ACC2713, for the diagnosis or treatment of disorders, in particular of neuropsychiatric disorders such as schizophrenia, depression or bipolar affective disorder.

10. Antibody which can be raised according to one of Claims 1 to 6, with the name 7B2, which can be produced using hybridoma cells which have been deposited under the number DSM ACC2714, for the diagnosis or treatment of disorders, in particular of neuropsychiatric disorders, in particular of neuropsychiatric disorders such as schizophrenia, depression or bipolar affective disorder.

11. Use of antibodies raised by the method according to Claims 1-6, or antibodies according to Claims 9 or 10, for the preparation of a pharmaceutical composition for the treatment of the neuropsychiatric disorders schizophrenia, depression or bipolar affective disorder, which pharmaceutical composition can be administered to a patient in such a form that it reaches the brain.

12. Use according to Claim 11, wherein the antibodies are linked to pharmaceutically active substances.

13. Use of misfolded proteins which can be isolated by a process in which
- brain fractions of patients with a neuropsychiatric disorder are purified in a targeted manner so as to concentrate post-translationally misfolded proteins, where during the purification process a purification step is carried out in the presence of ionic detergents at 0-10°C and the non-denatured misfolded proteins are pelleted under centrifugation conditions and which are recognized by the antibodies raised in accordance with Claim 1, for the preparation of a pharmaceutical composition for the treatment of the neuropsychiatric disorders schizophrenia, depression or bipolar affective disorder.

14. Use according to Claim 13, wherein the misfolded proteins can be assigned to one of the disorders schizophrenia, depression or bipolar affective disorder.

## Revendications

1. Procédé d'obtention d'anti-anticorps reconnaissant des protéines mal repliées pouvant être spécifiquement assignées à une maladie neuropsychiatrique telle la schizophrénie, la dépression ou les troubles affectifs bipolaires, dans lequel
- des fractions du cerveau de patients atteints d'une maladie neuropsychiatrique sont soumises à une épuration ciblée en vue d'obtenir une concentration de protéines post-translationnellement mal repliées, l'épuration comportant une étape de purification en présence de détergents ioniques à 0-10 °C et une pelletisation sous centrifugation des protéines mal repliées non dénaturées, et
- des anticorps sont produits avec les fractions épurées, et
- sont choisis, parmi ces anticorps, ceux qui se lient spécifiquement aux protéines mal repliées se trouvant dans les fractions épurées.

2. Procédé selon la revendication 1 pour lequel le détergent ionique mis en oeuvre pendant l'étape de purification est utilisé à une concentration comprise entre 0,2 et 2 %.

3. Procédé selon les revendications 1 ou 2 pour lequel le détergent ionique mis en oeuvre pendant l'étape de purification est du sarkosyl.

4. Procédé selon les revendications 1 à 3 dans lequel l'étape de purification avec un détergent ionique comporte une étape d'ultracentrifugation à au moins 100 000 x g.

5. Procédé selon les revendications 1 à 4 dans lequel est prévue, pendant l'épuration, une étape de purification avec des substances, éventuellement immobilisées, liant le feuillet ß, telles le rouge Congo, la thioflavine ou des peptides liant le feuillet ß.

6. Procédé selon les revendications 1 à 5 dans lequel est prévue, pendant l'épuration, une étape de digestion par protéase à une température de 0 - 10 °C.

7. Procédé pour la détection de marqueurs de maladies neuropsychiatriques telles la schizophrénie, la dépression ou les troubles affectifs bipolaires, dans lequel
- des fractions du cerveau de patients atteints d'une maladie neuropsychiatrique sont soumises, conformément à la revendication 1, à une épuration ciblée en vue d'obtenir une concentration de protéines post-translationnellement mal repliées,
- sont produits, avec les fractions épurées, des anticorps qui se lient spécifiquement aux protéines mal repliées se trouvant dans les fractions épurées, et
- des protéines qui ne sont présentes que chez les patients atteints sont détectées avec les anti-anticorps dans des homogénéisats de cerveau.

8. Utilisation comme marqueurs diagnostiques de maladies neuropsychiatriques de protéines mal repliées qui ont été, conformément à la revendication 1, concentrées dans les fractions du cerveau de patients atteints d'une maladie neuropsychiatrique puis extraites de ces fractions et isolées.

9. Anticorps produit selon l'une des revendications 1 à 6 désigné par 7B2 pouvant être produit à partir de cellules d'hybridomes déposées sous le numéro DSM ACC2713, en vue du diagnostic ou du traitement de maladies, notamment de maladies neuropsychiatriques telles la schizophrénie, la dépression ou les troubles affectifs bipolaires.

10. Anticorps produit selon l'une des revendications 1 à 6 désigné par 9C9 pouvant être produit à partir de cellules d'hybridomes déposées sous le numéro DSM ACC2714, en vue du diagnostic ou du traitement de maladies, notamment de maladies neuropsychiatriques telles la schizophrénie, la dépression ou les troubles affectifs bipolaires.

11. Utilisation d'anticorps produits par le procédé selon les revendications 1 à 6 ou d'anticorps selon les revendications 9 ou 10 pour la fabrication d'une composition pharmaceutique applicable à un patient sous une forme pouvant être transportée dans le cerveau en vue du traitement des maladies neuropsychiatriques schizophrénie, dépression, trouble affectif bipolaire.

12. Utilisation selon la revendication 11, les anticorps étant couplés à des substances pharmaceutiques actives.

13. Utilisation de protéines mal repliées pouvant être isolées par un procédé dans lequel
- des fractions du cerveau de patients atteints d'une maladie neuropsychiatrique sont soumises à une épuration ciblée pour obtenir une concentration de protéines post-translationnellement mal repliées, l'épuration comportant une étape de purification en présence de détergents ioniques à 0-10 °C et une pelletisation sous centrifugation des protéines mal repliées non dénaturées, et dans lequel les anti-anticorps produits selon la revendication 1 sont reconnus, ce procédé étant destiné à la fabrication d'une composition pharmaceutique pour le traitement des maladies neuropsychiatriques schizophrénie, dépression ou trouble affectif bipolaire.

14. Utilisation selon la revendication 13, les protéines mal repliées pouvant être assignées à l'une des maladies schizophrénie, dépression ou trouble affectif bipolaire.
